# EUROPEAN PATENT APPLICATION

(11) **EP 1 760 057 A1**
(43) Date of publication of application: **07.03.2007**
(21) Application number: 05752842.4
(22) Date of filing: 20.06.2005
(51) Int. Cl.: C07C 17/10, C07C 23/46, C07C 29/124, C07C 35/44, C07B 61/00

(54) **METHOD FOR PRODUCING POLYHALOGENATED DIAMANTANE AND DERIVATIVE THEREOF**

(30) Priority: 23.06.2004 JP 2004185590; 23.06.2004 JP 2004185591
(71) Applicant: TOKUYAMA CORPORATION, Tokuyama-shi, Yamaguchi-ken 745-8648 (JP)
(72) Inventor: MAEHARA, Takayuki, Shunan-shi, Yamaguchi 745-8648 (JP); YAMAGUCHI, Masao, Shunan-shi, Yamaguchi 745-8648 (JP)
(74) Representative: Ziebig, Marlene
(86) International application number: PCT/JP2005/011678
(87) International publication number: WO 2006/001398

(57) **Abstract**

The present invention discloses a method for producing a polyhalogenated diamantane, **characterized by** reacting a diamantane with a halosulfonic acid; and a method for producing a diamantanepolyol, **characterized by** reacting a polyhalogenated diamantane produced by the above-described method, with water in the presence of a water-soluble organic solvent and a carboxylic acid salt.

## Description

### Technical Field

The present invention relates to a method for producing a polyhalogenated diamantane or a diamantanepolyol, both useful, for example, as a raw material for production of functional material or electronic material.

### Background Art

Diamantane derivatives have a skeleton similar to that of adamantane derivatives. Since adamantane derivatives have a feature of superior heat resistance and high transparency, diamantane derivatives having a skeleton similar to that of adamantane derivatives are expected to be utilized as well, as a raw material for production of highly functional material (e.g. heat-resistant polymer) or electronic material (e.g. resist for semiconductor, requiring transparency). Of the diamantane derivatives, polyhalogenated diamantanes are important compounds as a starting raw material for production of various diamantane derivatives.

The method for production of polyhalogenated diamantane includes a method of reacting diamantane with bromine using an aluminum bromide catalyst (Collection of Czechosolvak Chemical Communications, 1983, Vol. 48, p. 1162; Journal of the organic Chemistry, 1974, Vol. 39, p. 2995). In this method, monobromodiamantane, dibromodiamantane, tribromodiamantane, etc. are produced.

In the above production method, the dibromodiamantane obtained is a mixture of isomers in which bromine atoms are bonded to the 4,9-, 1,6- or 1,4-positions of diamantane. These isomers are similar in chemical and physical properties, making difficult the separation of individual isomers. Further in the production method, bromine is used in a large amount, making high the production cost.

With respect to the method for production of monochlorodiamantane, there is known a method of reacting diamantane with chlorosulfonic acid at -10°C to -5°C (Journal of the Chemical Society Perkin Transaction 1, 1972, p. 2691). By this method, there is produced a mixture of 1-chlorodiamantane and 4-chlorodiamantane.

With respect to the method for production of dichlorodiamantane, there is a method of producing 4,9-dichlorodiamantane by using 4,9-diamantanediol as a starting raw material. However, the present inventors do no know any report regarding the method for production of polychlorodiamantane by using diamantane as a starting raw material.

Use of halosulfonic acid in production of polyhalogenated diamantane is preferred for reduced production cost because halosulfonic acid is relatively inexpensive. However, the present inventors do no know any report regarding the method for production of dihalogenated diamantane or trichlorodiamantane by reacting diamantane (as a starting raw material) with a halosulfonic acid.

As described above, diamantane is not used as a raw material in the conventional methods for production of polyhalogenated diamantane. Further, in the conventional production methods, no polyhalogenated diamantane of single structure is obtained. Therefore, in order to obtain a polyhalogenated diamantane of single structure, excessive purification is needed, which is not advantageous industrially for the cost incurred. Also, when a mixture of polyhalogenated diamantanes in each of which the substitution positions of halogen atom are different, is used as a raw material to produce corresponding diamantane derivatives and these diamantane derivatives are used as an electronic material, etc., the proportions of individual diamantane derivatives each different in substitution positions may fluctuate in each produced electronic material, etc. In such a case, the obtained electronic material, etc. may not have constant properties.

Meanwhile, of diamantane derivatives, diamantanepolyols are important compounds as a starting raw material for production of various diamantane derivatives.

The method for production of diamantanepolyol includes a method of oxidizing diamantane with 96% sulfuric acid (Journal of the Chemical Society Perkin Transaction 1, 1972, p. 2691). Also, there is a method of reacting dibromodiamantane with 65% nitric acid to obtain a diamantanediol (Collection of Czechosolvak Chemical Communications, 1983, Vol. 48, p. 1162). In the former method, the selectivity is low and diamantanone, etc. are formed besides the intended diamantanediol, and 4,9-diamantanediol is formed in an amount of only 5%.

In the latter method, a diamantanediol is obtained in 53% yield. However, in this method, replacement of dibromodiamantane with dichlorodiamantane (which is easy to handle industrially) is considered to be difficult. The first reason therefor is that there is presently no appropriate method for production of dichlorodiamantane. As the second reason, when presumed from a common knowledge of chemistry that the reactivity of dichlorodiamantane will be low as compared with that of dibromodiamantane, it is considered that even if it is tried to convert a dichlorodiamantane to a diamantanediol under the same conditions, the reaction does not proceed substantially and no diamantanediol is obtained. In fact, there is no report of replacing dibromoamantane with dichlorodiamantane.

Further, there is no report of converting a trihalogenated diamantane into a diamantanetriol.

Thus, the above-mentioned methods for production of diamantanepolyols are not advantageous industrially because the yield of reaction is low and the kinds of polyhalogenated diamantanes usable are small. Therefore, there is desired a method capable of producing a diamantanepolyol inexpensively and at a high yield.

### Disclosure of the Invention

The present inventors made a study in order to solve the above problems. As a result, the present inventors found that a polyhalogenated diamantane can be obtained at a high purity and inexpensively by reacting a diamantane with a halosulfonic acid. The present inventors further found that a diamantanepolyol can be obtained by reacting a polyhalogenated diamantane with water in the presence of a water-soluble organic solvent and a carboxylic acid salt. The present invention has been completed based on these findings.

The first object of the present invention lies in providing a method for producing a polyhalogenated diamantane from a diamantane used as a starting raw material, at a high purity and inexpensively.

The second object of the present invention lies in providing a method for producing a diamantanepolyol from the polyhalogenated diamantane produced by the above method, inexpensively and at a high yield.

The present invention lies in a method for producing a polyhalogenated diamantane, characterized by mixing and reacting a diamantane with a halosulfonic acid to obtain a polyhalogenated diamantane at a high purity and inexpensively. In this reaction system, concentrated sulfuric acid or an inorganic salt may be added. Further, the halosulfonic acid may be added into the reaction system in a plurality of fractions.

The present invention further lies in a method for producing a diamantanepolyol by reacting a polyhalogenated diamantane with water in the presence of a water-soluble organic solvent and a carboxylic acid salt to produce a diamantanepolyol.

According to the present invention, a polyhalogenated diamantane can be produced from a diamantane at a high purity and inexpensively. In general, diamantanes are compounds which have many crosslinkage positions and many bridgehead positions (the positions of tertiary carbons in diamantane skeleton); when these compounds are polyhalogenated, there can ordinarily be obtained a mixture of a plurality of polyhalogenated diamantanes each different in the substitution positions of halogen. In the present invention, it is possible to introduce halogen atoms selectively into the particular bridgehead positions of the diamantane used as a raw material. Therefore, the production method of the present invention is very useful as a method for industrially producing a polyhalogenated diamantane which is important as a raw material for synthesis of various diamantane derivatives including a functional polymer.

Further, according to the present invention, a diamantanepolyol can be obtained inexpensively and at a high yield, by using the above-produced polyhalogenated diamantane as a raw material. It is known generally that chlorinated compounds, as compared with brominated compounds or iodinated compounds, are inexpensive but are low in reactivity. However, according to the present invention, an intended product can be obtained at a high yield even though a polychlorinated diamantane is used as a raw material. The production method of the present invention is very useful as a method for industrially producing a diamantanepolyol which is important, for example, as a raw material for synthesis of various diamantane derivatives including a functional polymer.

### Brief Description of the Drawings

Fig. 1 is an NMR spectrum of the 4,9-dichlorodiamantane obtained in Example 5.
Fig. 2 is a ¹³C-NMR spectrum of the 4,9-dichlorodiamantane obtained in Example 5.
Fig. 3 is an NMR spectrum of the 1,4,9-trichlorodiamantane obtained in Example 8.
Fig. 4 is a ¹³C -NMR spectrum of the 1,4,9-trichlorodiamantane obtained in Example 8.
Fig. 5 is an NMR spectrum of the dibromodiamantane obtained in Comparative Example 1.
Fig. 6 is an NMR spectrum of the 4,9-diamantanediol obtained in Example 17.
Fig. 7 is a ¹³C-NMR spectrum of the 4,9-diamantanediol obtained in Example 17.
Fig. 8 is an NMR spectrum of the 1,4,9-diamantanetriol obtained in Example 23.
Fig. 9 is a ¹³C-NMR spectrum of the 1,4,9-diamantanetriol obtained in Example 23.

### Best Mode for Carrying Out the Invention

### Production of Polyhalogenated Diamantane

In the method of the present invention for production of polyhalogenated diamantane, a diamantane is reacted with a halosulfonic acid to obtain a polyhalogenated diamantane.

In-depth description is made below on the raw materials of reaction, reaction conditions and procedure used in the production method of the present invention, as well as on the product obtained, etc.

As the diamantane used as a raw material in the production method of the present invention, any diamantane can be used as long as it has a diamantane skeleton. A diamantane represented by the following formula (1) 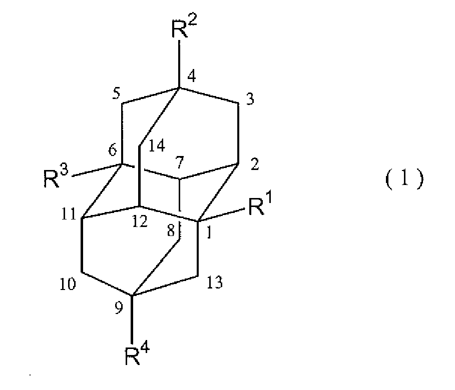 (wherein R¹ to R⁴ are each independently a hydrogen atom or an alkyl group having 1 to 5 carbon atoms), is preferred for the importance of the industrial use, the good availability, etc.

In the above formula (1), R¹ to R⁴ are each a hydrogen atom or an alkyl group having 1 to 5 carbon atoms. As the alkyl group, there can be specifically mentioned methyl group, ethyl group, propyl group, butyl group and pentyl group and, among them, methyl group is particularly preferred for the reasons mentioned above.

As preferred specific examples Of the diamantane represented by the above formula (1), there can be mentioned diamantane, 1-methyldiamantane, 1-ethyldiamantane, 4-methyldiamantane, 4-ethyldiamantane, 1,4-dimethyldiamantane, 1,4-diethyldiamantane, 1,6-dimethyldiamantane, 1,6-diethyldiamantane, 4,9-dimethyldiamantane and 4,9-diethyldiamantane.

The halosulfonic acid used in the production method of the present invention as another raw material is a compound represented by the following formula (2)

XSO₃H (2)

(wherein X is a fluorine atom, a chlorine atom, a bromine atom or an iodine atom).

As preferred specific examples of the halosulfonic acid represented by the formula (2), there can be mentioned chlorosulfonic acid, bromosulfonic acid and iodosulfonic acid. Chlorosulfonic acid is preferred particularly for the low cost.

The amount of the halosulfonic acid used is preferably determined in view of a balance between its property of acting also as a reaction solvent and moreover giving an increased reaction rate and the easy post-treatment after the completion of reaction.

That is, when it is aimed to produce a dihalogenated diamantane, the use amount of halosulfonic acid is preferably 2 to 50 mols, more preferably 3 to 25 mols, particularly preferably 4 to 10 mols per mol of the diamantane used.

When it is aimed to produce a trihalogenated diamantane, the use amount of halosulfonic acid is preferably 3 to 100 mols, more preferably 4 to 50 mols, particularly preferably 6 to 40 mols per mol of the diamantane used.

When a diamantane is halogenated, the dihalogenated diamantane first formed dissolves in the halosulfonic acid used, to a certain extent and, therefore, the reaction proceeds further from dihalogenation to trihalogenation; as a result, there is a case that a trihalogenated diamantane is formed. Therefore, when it is aimed to produce a dihalogenated diamantane, it is preferred to allow concentrated sulfuric acid (which is a poor solvent to the dihalogenated diamantane) to be present in the reaction system.

When concentrated sulfuric acid is allowed to be present in the reaction system, the dihalogenated diamantane formed by the reaction of diamantane with halosulfonic acid separates out from the reaction mixture. As a result, the formation of trihalogenated diamantane is suppressed, and a dihalogenated diamantane can be produced at a high selectivity. Further, when concentrated sulfuric acid is added into a reactor before addition of diamantane into the reactor, there is also an advantage that the breakage of reactor at diamantane addition is prevented.

Mixing of concentrated sulfuric acid may be conducted by any method. Ordinarily, it is preferred to add a halosulfonic acid into a suspension between diamantane and concentrated sulfuric acid. The amount of concentrated sulfuric acid used is not particularly restricted. However, in view of the large amount of the dihalogenated diamantane obtained and the easiness of post-treatment, the use amount of concentrated sulfuric acid is preferably 0.1 to 20 parts by mass, particularly preferably 0.5 to 10 parts by mass per part by mass of the diamantane used.

Incidentally, concentrated sulfuric acid may be added not only in production of dihalogenated diamantane but also in production of trihalogenated diamantane. As described above, prior addition of concentrated sulfuric acid into reactor can lessen the breakage of reactor taking place in addition of diamantane into reactor. In this case, the amount of concentrated sulfuric acid used is the same as in the case of dihalogenated diamantane.

When a halogenation reaction is conducted by adding concentrated sulfuric acid into the reaction system, the water contained in the concentrated sulfuric acid reacts with a halosulfonic acid and consequently the halosulfonic acid may be consumed without reacting with a diamantane. In this case, the amount of the halosulfonic acid used refers to an amount obtained by subtracting the amount of the halosulfonic acid decomposed by the water contained in concentrated sulfuric acid.

When halogenation of diamantane is conducted by adding a halosulfonic acid into the reaction system, it is possible to add the halosulfonic acid into the reaction system at one time, or add the halosulfonic acid taking a relatively long time, continuously or intermittently in small amounts. However, it is preferred to divide the halosulfonic acid in a plurality of fractions and add each fraction stepwise when an appropriate time has passed. By thus adding a plurality of fractions intermittently, the run-away of reaction can be prevented and an intended halogenated diamantane can be produced selectively at a high purity. When the concentrated sulfuric acid is present in the reaction system, there is a case that the water contained in the concentrated sulfuric acid reacts with the halosulfonic acid violently, inviting the run-away of reaction. In this case as well, the run-away of reaction can be prevented by adding the halosulfonic acid in fractions.

Division of halosulfonic acid may be conducted in any of two or more fractions and is conducted preferably in 2 to 5 fractions. With respect to the time intervals of addition of second and later fractions, there is no restriction. For example, it is preferred that the progress of halogenation is examined by gas chromatography (hereinafter abbreviated to GC) or the like and, when substantial termination of the progress of reaction has been confirmed thereby, a fresh fraction is added to restart the reaction.

There is no restriction, either, as to the amounts of halosulfonic acid divided. For example, when the halosulfonic acid is used in an amount of 5 mols per mol of diamantane, the division of halosulfonic acid may be such that 2 mols of the halosulfonic acid is used at the start of reaction and, when substantial termination of the progress of reaction has been confirmed by GC analysis, the remaining 3 mols are added.

In the present invention, it is preferred that an inorganic salt is present in the reaction system. When an inorganic salt is present in the reaction system, the polyhalogenated diamantane obtained has a higher purity and the coloring thereof, etc. are alleviated. As the inorganic salt, a known substance can be used with no restriction. For good availability, there can be mentioned metal chlorides such as sodium chloride, potassium chloride, magnesium chloride, calcium chloride and the like; metal bromides such as sodium bromide, potassium bromide, magnesium bromide, calcium bromide and the like; metal iodides such as sodium iodide, potassium iodide, magnesium iodide, calcium iodide and the like; metal carbonates such as lithium carbonate, sodium carbonate, magnesium carbonate, potassium carbonate, calcium carbonate and the like; sulfates such as lithium sulfate, sodium sulfate, magnesium sulfate, potassium sulfate, calcium sulfate and the like; and so forth. Of these, metal chlorides and sulfates are preferred because the polyhalogenated diamantane obtained has a higher purity and is significantly reduced in coloring, and sodium chloride and sodium sulfate are preferred particularly.

With respect to the addition amount of inorganic salt, there is no particular restriction. However, too large an addition amount results in a lower reaction rate and too small an addition amount results in a reduced addition effect. Therefore, the addition amount of inorganic salt is preferably 0.01 to 50 mols, particularly preferably 0.05 to 10 mols per mol of the diamantane used.

The addition timing of the inorganic salt is not restricted particularly. However, the inorganic salt is preferred to be added ordinarily at the start of the reaction, for easy operation.

There is no particular restriction as to the temperature of the reaction between diamantane and halosulfonic acid. The reaction temperature may be selected appropriately depending upon the polyhalogenated diamantane to be obtained. Ordinarily, when the reaction temperature is lower, the reaction rate is small and the solubilities of diamantane and formed halogenated diamantane in reaction mixture are low; therefore, the number of halogen atoms introduced into diamantane is small. When the reaction temperature is higher, the situation is opposite to the above and the number of halogen atoms introduced into diamantane is large. Considering from these matters, the reaction temperature is generally in a range of 0 to 100°C.

When it is aimed to produce a dihalogenated diamantane selectively, the reaction temperature is preferably 0 to 60°C, more preferably 5 to 50°C.

When it is aimed to produce a trihalogenated diamantane selectively, the reaction temperature is preferably 20 to 100°C, more preferably 25 to 90°C.

There is no particular restriction as to the time of reaction. However, the reaction time is preferred to be 4 to 48 hours ordinarily. With such a reaction time, a sufficient conversion is obtained.

In the present invention, an organic solvent may be added into the reaction system. As the organic solvent, there can be used those having a low reactivity with halosulfonic acid, concentrated sulfuric acid, etc. Specifically, there can be mentioned, for example, halogenated aliphatic hydrocarbons such as methylene chloride, chloroform, carbon tetrachloride and the like.

The apparatus used in the present invention is preferably an apparatus having such a structure that the inside can be beforehand purged sufficiently with an inert gas (e.g. nitrogen) and dried and, during the reaction, an inert gas (e.g. nitrogen) can be passed trough the inside, in order to prevent the reaction of halosulfonic acid with water and resultant decomposition of halosulfonic acid.

As to the post-treatment of reaction products, conducted after the completion of reaction, the following method can be mentioned, for example. That is, the reaction mixture is cooled to room temperature; then, while the reaction mixture is being cooled to 30°C or less, water is dropped into the reaction mixture to decompose the remaining halosulfonic acid. Then, there is added an organic solvent (e.g. dichloromethane or chloroform) capable of dissolving the formed polyhalogenated diamantane, to extract the polyhalogenated diamantane. The organic layer obtained by extraction is washed with an aqueous solution (e.g. a basic aqueous solution) until the organic layer becomes neutral. After confirming that the organic layer is neutral, the organic layer is concentrated, whereby can be obtained a crude polyhalogenated diamantane. Ordinarily, this crude substance contains an intended polyhalogenated diamantane in an amount of 80 mass % or more.

When the polyhalogenated diamantane is sparingly soluble in the organic solvent and extraction by the organic solvent is difficult, the crude substance may be separated from the reaction mixture by filtration or centrifugation.

The crude polyhalogenated diamantane isolated by the above post-treatment can be used per se in various usages. By applying, to the crude substance, an adsorption treatment such as active carbon treatment, silica treatment, alumina treatment or the like, various impurities contained in the crude substance are removed; thereby, the crude substance is purified to a higher purity and also decolored (the coloration caused by impurities is removed).

Besides the above treatment, there can be used a known purification method such as recrystallization, purification by sublimation, re-slurrying treatment or the like, whereby an intended product of higher purity can be obtained.

In the case of the present invention, the re-slurrying treatment is a method of adding, to the crude polyhalogenated diamantane, an organic solvent capable of selectively dissolving the impurities contained in the crude substance, to prepare a slurry, then filtering the slurry, followed by drying, to obtain a polyhalogenated diamantane of higher purity.

Incidentally, measurement of purity can be made by GC.

### (Dihalogenated Diamantane)

The dihalogenated diamantane obtained by the above production method has the bridgeheads of diamantane at six positions and, therefore, has three kinds of isomers.

Firstly, when one of the compounds represented by the above-shown formula (1), such as diamantane, 1-methyldiamantane, 1-ethyldiamantane, 1,6-dimethyldiamantane or 1,6-diethyldiamantane is used as a raw material and halogenated, there can be obtained a 4,9-dihalogenated diamantane represented by the following formula (3), having halogen atoms introduced into the 4,9-positions of diamantane skeleton. 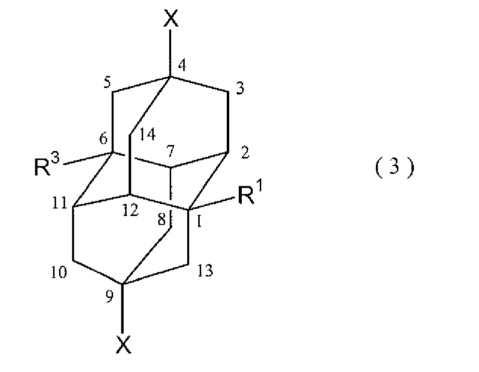 [wherein R¹ and R³ have each the same definition as given in the formula (1) and X is a halogen atom such as fluorine, chlorine, bromine or iodine.]

As the 4,9-dihalogenated diamantane represented by the formula (3), there can be mentioned, for example, 4,9-dichlorodiamantane, 4,9-dibromodiamantane, 4,9-diiododiamantane, 1-methyl-4,9-dichlorodiamantane, 1-methyl-4,9-dibromodiamantane, 1-methyl-4,9-diiododiamantane, 1-ethyl-4,9-dichlorodiamantane, 1-ethyl-4,9-dibromodiamantane, 1-ethyl-4,9-diiododiamantane, 1,6-dimethyl-4,9-dichlorodiamantane, 1,6-dimethyl-4,9-dibromodiamantane, 1,6-dimethyl-4,9-diiododiamantane, 1,6-diethyl-4,9-dichlorodiamantane, 1,6-diethyl-4,9-dibromodiamantane and 1,6-diethyl-4,9-diiododiamantane.

Secondly, when one of the compounds represented by the above-shown formula (1), such as diamantane, 4-methyldiamantane, 4-ethyldiamantane, 4,9-dimethyldiamantane or 4,9-diethyldiamantane is used as a raw material and halogenated, there can be obtained a 1,6-dihalogenated diamantane represented by the following formula (4), having halogen atoms introduced into the 1,6-positions of diamantane skeleton. 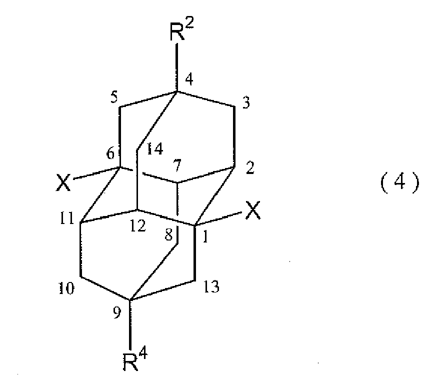 [wherein R² and R⁴ have each the same definition as given in the formula (1) and X is a halogen atom such as fluorine, chlorine, bromine or iodine.]

As the 1,6-dihalogenated diamantane represented by the formula (4), there can be mentioned, for example, 1,6-dichlorodiamantane, 1,6-dibromodiamantane, 1,6-diiododiamantane, 4-methyl-1,6-dichlorodiamantane, 4-methyl-1,6-dibromodiamantane, 4-methyl-1,6-diiododiamantane, 4-ethyl-1,6-dichlorodiamantane, 4-ethyl-1,6-dibromodiamantane, 4-ethyl-1,6-diiododiamantane, 4,9-dimethyl-1,6-dichlorodiamantane, 4,9-dimethyl-2,6-dibromodiamantane, 4,9-dimethyl-1,6-diiododiamantane, 4,9-diethyl-1,6-dichlorodiamantane, 4,9-diethyl-1,6-dibromodiamantane and 4,9-diethyl-1,6-diiododiamantane.

Thirdly, when one of the compounds represented by the above-shown formula (1), such as diamantane, 1-methyldiamantane, 1-ethyldiamantane, 4-methyldiamantane, 4-ethyldiamantane, 1,4-dimethyldiamantane or 1,4-diethyldiamantane is used as a raw material and halogenated, there can be obtained a 1,4-dihalogenated diamantane represented by the following formula (5), having halogen atoms introduced into the 1,4-positions of diamantane skeleton. 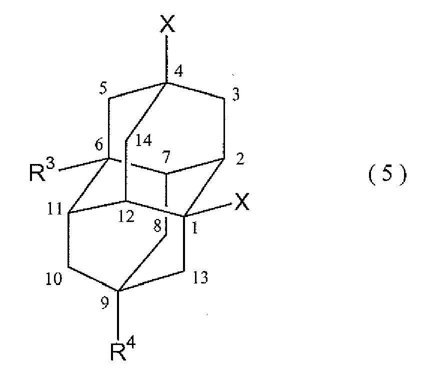 [wherein R³ and R⁴ have each the same definition as given in the formula (1) and X is a halogen atom such as fluorine, chlorine, bromine or iodine.]

As the 1,4-dihalogenated diamantane represented by the formula (5), there can be mentioned, for example, 1,4-dichlorodiamantane, 1,4-dibromodiamantane, 1,4-diiododiamantane, 6-methyl-1,4-dichlorodiamantane, 6-methyl-1,4-dibromodiamantane, 6-methyl-1,4-diiododiamantane, 6-ethyl-1,4-dichlorodiamantane, 6-ethyl-1,4-dibromodiamantane, 6-ethyl-1,4-diiododiamantane, 9-methyl-1,4-dichlorodiamantane, 9-methyl-1,4-dibromodiamantane, 9-methyl-1,4-diiododiamantane, 9-ethyl-1,4-dichlorodiamantane, 9-ethyl-1,4-dibromodiamantane, 9-ethyl-1,4-diiododiamantane, 6,9-dimethyl-1,4-dichlorodiamantane, 6,9-dimethyl-1,4-dibromodiamantane, 6,9-dimethyl-1,4-diiododiamantane, 6,9-diethyl-1,4-dichlorodiamantane, 6,9-diethyl-1,4-dibromodiamantane and 6,9-diethyl-1,4-diiododiamantane.

When diamantane is used as a raw material and is halogenated according to the production method of the present invention, there can be selectively obtained a 4,9-dihalogenated diamantane which is one of three kinds of isomers. In this case, the selectivity is ordinarily 80% or more.

### (Trihalogenated Diamantane)

In production of trihalogenated diamantane, two kinds of trihalogenated diamantanes are formed.

Firstly, when one of the compounds represented by the above-shown formula (1), that is, diamantane, 1-methyldiamantane or 1-ethyldiamantane is used as a raw material and halogenated, there can be obtained a 1,4,9-trihalogenated diamantane represented by the following formula (6), having halogen atoms bonded to the carbon atoms of 1,4,9-positions of diamantane skeleton. 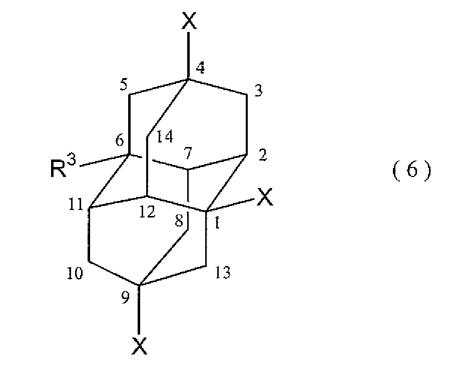 [wherein R³ has the same definition as given in the formula (1), and X is a halogen atom such as fluorine, chlorine, bromine or iodine.]

As specific examples of the 1,4,9-trihalogenated diamantane represented by the formula (6), there can be mentioned 1,4,9-trichlorodiamantane, 1,4,9-tribromodiamantane, 1,4,9-triiododiamantane, 6-methyl-1,4,9-trichlorodiamantane, 6-methyl-1,4,9-tribromodiamantane, 6-methyl-1,4,9-triiododiamantane, 6-ethyl-1,4,9-trichlorodiamantane, 6-ethyl-1,4,9-tribromodiamantane and 6-ethyl-1,4,9-triiododiamantane.

Secondly, when one of the compounds represented by the above-shown formula (1), that is, diamantane, 4-methyldiamantane or 4-ethyldiamantane is used as a raw material and halogenated, there can be obtained a 1,4,6-trihalogenated diamantane represented by the following formula (7), having halogen atoms bonded to the carbon atoms of 1,4,6-positions of diamantane skeleton. 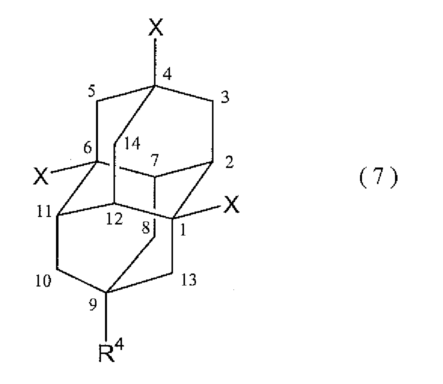 [wherein R⁴ has the same definition as given in the formula (1), and X is a halogen atom such as fluorine, chlorine, bromine or iodine.]

As specific examples of the 1,4,6-trihalogenated diamantane represented by the formula (7), there can be mentioned 1,4,6-trichlorodiamantane, 1,4,6-tribromodiamantane, 1,4,6-triiododiamantane, 9-methyl-1,4,6-trichlorodiamantane, 9-methyl-1,4,6-tribromodiamantane, 9-methyl-1,4,6-triiododiamantane, 9-ethyl-1,4,6-trichlorodiamantane, 9-ethyl-1,4,6-tribromodiamantane and 9-ethyl-1,4,6-triiododiamantane.

### Production of Diamantanepolyol

In the present method for production of diamantanepolyol, it is preferred that the polyhalogenated diamantane obtained by the above-mentioned method for production of polyhalogenated diamantane is reacted with water in the presence of a water-soluble organic solvent and a carboxylic acid salt. In-depth description is made below on the raw material, catalyst, etc., reaction conditions and reaction procedure used in the present method for production of diamantanepolyol, as well as on the formed product, etc.

### (Polyhalogenated Diamantane)

In the present invention, the polyhalogenated diamantane used as a raw material may be a polyhalogenated diamantane produced by any method. However, it is preferred to use a dihalogenated diamantane or a trihalogenated diamantane, which is produced based on one of the above-mentioned production methods or one of the Production Examples mentioned later.

### (Water-Soluble Organic Solvent)

As the water-soluble organic solvent used in the present invention, there can be used any known water-soluble organic solvent miscible with water at normal temperature. As specific examples thereof, there can be mentioned methanol, ethanol, 2-propanol, 2-methoxyethanol, 2-ethoxyethanol, acetone, acetonitrile, N,N-dimethylformamide, formamide, triethylamine, pyridine and N,N,N,N-tetramethylethylenediamine. N,N-dimethylformamide is preferred particularly because it enables a rapid reaction and is inexpensive.

The amount of the water-soluble organic solvent used is not particularly restricted. However, the use amount is preferably determined in view of a balance between the level of reaction yield and the smoothness of reaction.

The use amount of water-soluble organic solvent is preferably 1 to 100 mols, more preferably 2 to 50 mols per mol of dihalogenated diamantane, when the polyhalogenated diamantane used is a dihalogenated diamantane. When there is used a trihalogenated diamantane, the use amount of water-soluble organic solvent is preferably 2 to 200 mols, more preferably 3 to 100 mols per mol of trihalogenated diamantane.

### (Water)

In the present invention, the water used has a role of hydrolyzing the polyhalogenated diamantane used as a raw material. The use amount thereof is preferably determined in view of a balance between the rapidness of reaction and the level of recovery ratio. When the polyhalogenated diamantane used is a dihalogenated diamantane, the use amount of water is preferably 5 to 500 mols, more preferably 20 to 400 mols per mol of dihalogenated diamantane. When there is used a trihalogenated diamantane, the use amount of water is preferably 5 to 1,000 mols, more preferably 30 to 800 mols per mol of trihalogenated diamantane.

### (Carboxylic Acid Salt)

Carboxylic acid salt has, in the molecule, a cation capable of bonding with the halogen atom possessed by polyhalogenated diamantane. Carboxylic acid salt captures the hydrogen halide formed when a polyhalogenated diamantane is reacted and converted into a diamantanepolyol.

As the carboxylic acid salt, any known carboxylic acid salt can be used. Specifically, there can be mentioned alkali metal formates such as lithium formate, sodium formate, potassium formate and the like; alkaline earth metal formates such as magnesium formate, calcium formate, barium formate and the like; alkali metal acetates such as lithium acetate, sodium acetate, potassium acetate and the like; alkaline earth metal acetates such as magnesium acetate, calcium acetate, barium acetate and the like; and so forth. Of these, preferred are lithium acetate, sodium acetate and potassium acetate in view of a balance between the availability and the reactivity. These carboxylic acid salts may be used in admixture of two or more kinds.

The amount of the carboxylic acid salt used is preferably determined in view of a balance between the yield of reaction and the level of reactivity. The use amount of carboxylic acid salt is preferably 2 to 10 mols, more preferably 2 to 5 mols per mol of dihalogenated diamantane, when the polyhalogenated diamantane used is a dihalogenated diamantane. When there is used a trihalogenated diamantane, the use amount of carboxylic acid salt is preferably 3 to 15 mols, more preferably 3 to 7 mols per mol of trihalogenated diamantane.

There is no particular restriction as to the reaction temperature. However, too low a reaction temperature results in slow proceeding of reaction and too high a reaction temperature results in inferior operation. Therefore, the reaction temperature is preferably 100 to 200°C, more preferably 120 to 180°C.

There is no particular restriction as to the reaction time. Ordinarily, a reaction time of 3 to 48 hours can give a sufficient conversion.

Preferably, the reaction is conducted under pressure in a closed system such as autoclave or the like in order to prevent the vaporization of the solvent used and keep the reaction temperature in the above-mentioned range. The reaction pressure differs depending upon the reaction temperature but ordinarily is preferred to be 0.2 to 0.8 MPa.

When the reaction is conducted using an autoclave, there is a case that the polyhalogenated diamantane having a sublimating property separates to deposit on the upper inner wall of autoclave, which is relatively low temperature and is unable to take part in the reaction. In the present invention, however, the water-soluble organic solvent (which is being refluxed) dissolves the polyhalogenated diamantane adhering onto the upper inner wall and returns it into the reaction mixture, whereby there is no stoppage of reaction.

When the dihalogenated diamantane represented by the formula (3) is used as a raw material, there is obtained a 4,9-diamantanediol represented by the following formula (8), of the diamantanepolyols obtained by the above reaction. 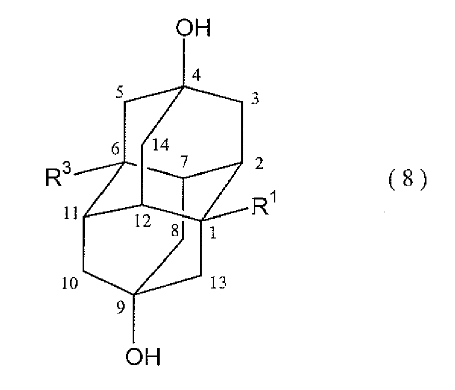 [wherein R¹ and R³ have each the same definition as given in the formula (1).]

As preferred specific examples of the 4,9-diamantanediol represented by the formula (6), there can be mentioned 4,9-diamantanediol, 1-methyl-4,9-diamantanediol, 1-ethyl-4,9-diamantanediol, 1,6-dimethyl-4,9-diamantanediol and 1,6-diethyl-4,9-diamantanediol.

When the dihalogenated diamantane represented by the formula (4) is used as a raw material, there is obtained a 1,6-diamantanediol represented by the following formula (9). 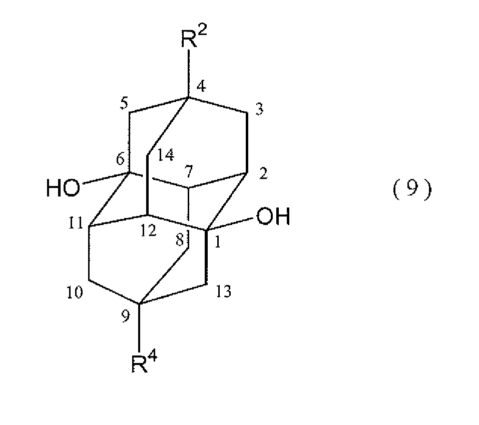 [wherein R² and R⁴ have each the same definition as given in the formula (1).]

As preferred specific examples of the 1,6-diamantanediol represented by the formula (9), there can be mentioned 1,6-diamantanediol, 4-methyl-1,6-diamantanediol, 4-ethyl-1,6-diamantanediol, 4,9-dimethyl-1,6-diamantanediol and 4,9-diethyl-1,6-diamantanediol.

When the dihalogenated diamantane represented by the formula (5) is used as a raw material, there is obtained a 1,4-diamantanediol represented by the following formula (10). 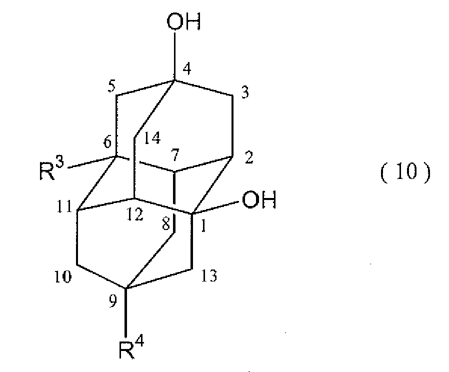 [wherein R³ and R⁴ have each the same definition as given in the formula (1).]

As preferred specific examples of the 1,4-diamantanediol represented by the formula (10), there can be mentioned 1,4-diamantanediol, 6-methyl-1,4-diamantanediol, 6-ethyl-1,4-diamantanediol, 9-methyl-1,4-diamantanediol., 9-ethyl-1,4-diamantanediol, 6,9-dimethyl-1,4-diamantanediol and 6,9-diethyl-1,4-diamantanediol.

When the trihalogenated diamantane represented by the formula (6) is used as a raw material, there is obtained a 1,4,9-diamantanetriol represented by the following formula (11). 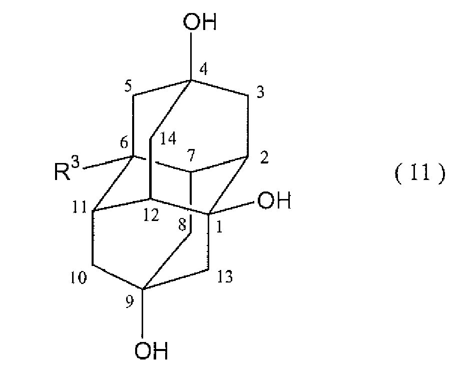 [wherein R³ has the same definition as given in the formula (1).]

As preferred specific examples of the 1,4,9-diamantanetriol represented by the formula (11), there can be mentioned 1,4,9-diamantanetriol, 6-methyl-1,4,9-diamantanetriol and 6-ethyl-1,4,9-diamantanetriol.

When the trihalogenated diamantane represented by the formula (7) is used as a raw material, there is obtained a 1,4,6-diamantanetriol represented by the following formula (12). 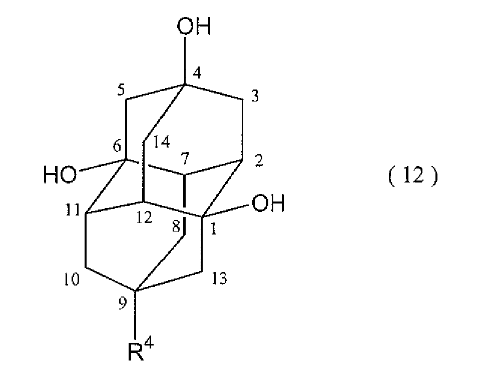 [wherein R⁴ has the same definition as given in the formula (1).]

As preferred specific examples of the 1,4,6-diamantanetriol represented by the formula (12), there can be mentioned 1,4,6-diamantanetriol, 9-methyl-1,4,6-diamantanetriol and 9-ethyl-1,4,6-diamantanetriol.

As to the method for isolating the diamantanepolyol formed by the above reaction, there is no particular restriction. However, the isolation can be conducted, for example, by the following method. That is, after the completion of the reaction, the reaction mixture is cooled to room temperature and the pressure inside the autoclave is returned to normal pressure. Then, the solid separated out is separated by a means such as filtration, centrifugation or the like to obtain the solid. The solid ordinarily contains an alkali metal halide formed as a by-product by the reaction between the hydrogen halide formed by hydrolysis of polyhalogenated diamantane and the carboxylic acid salt. The alkali metal halide can be removed by washing the solid with water. Thereby can be obtained a crude diamantanepolyol (the conversion relative to the raw material dihalogenated diamantane is ordinarily 95% or more). Alternatively, there can be obtained a crude diamantanepolyol by concentrating the reaction mixture, extracting the concentrate with an alcohol such as butanol or the like, and concentrating the extract.

There is no particular restriction as to the method for purifying the crude diamantanepolyol isolated as above. However, the following method is preferred.

First, to the crude diamantanepolyol is added an organic solvent (a poor solvent) which dissolves impurities in the crude diamantanepolyol (the by-products formed in the reaction, unreacted precursors, and other substances used in the reaction, other than intended product) but does not dissolve or sparingly dissolves the diamantanepolyol, that is, an organic solvent (hereinafter referred to also as re-slurrying solvent) which selectively dissolves the impurities contained in the crude diamantanepolyol, to form a slurry. Then, the slurry is filtered, the filtrate is removed, and the crude substance is dried. By this operation, there can be obtained a diamantanepolyol of high purity (which ordinarily contains a diamantanepolyol in an amount of 95 mol % or more) (this treatment is hereinafter referred to also as re-slurrying treatment).

As the organic solvent used in the above re-slurrying treatment, there are preferred, for the high effect of impurities removal, halogenated aliphatic hydrocarbons such as methylene chloride, chloroform, carbon tetrachloride and the like; aliphatic hydrocarbons such as hexane, heptane, octane and the like; halogenated aromatic hydrocarbons such as chlorobenzene, bromobenzene, dichlorobenzene, dibromobenzene and the like; ethers such as diethyl ether, diisopropyl ether, di-tert-butyl ether and the like; esters such as methyl acetate, ethyl acetate, propyl acetate, isopropyl acetate, butyl acetate, iso-butyl acetate and the like; ketones such as acetone, methyl ethyl ketone, methyl iso-butyl ketone and the like; cyano compounds such as acetonitrile and the like; and so forth. Regarding the re-slurrying treatment, there is a method of adding an organic solvent, once heating the solution, then cooling it to room temperature or lower, and filtering it. This method is preferred because the diamantanepolyol can be obtained in a larger amount.

In addition to the above re-slurrying treatment, there can be conducted an adsorption treatment such as active carbon treatment, silica treatment, alumina treatment or the like; thereby, various impurities can be removed, a higher purity is achieved, and the extent of decolorization (removal of the coloring caused by impurities) is enhanced. Besides such treatments, purification by known method such as recrystallization, sublimation purification or the like can as well give an intended product of higher purity.

Incidentally, the purity of intended product can be confirmed by gas chromatography (GC).

The present invention is described more specifically below by way of Examples. However, the present invention is in no way restricted by these Examples.

### Examples

### Example 1

40 g (2 times the mass of diamantane) of concentrated sulfuric acid and 1.5 g (0.0106 mol, 0.1 time the mol of diamantane) of sodium sulfate were placed in a 500-ml, four-necked flask in a nitrogen current. Further, 20 g (0.106 mol) of diamantane was added, and they were stirred for 10 minutes while being kept in a suspension state at about 20°C. Thereto was gradually added 24.7 g (0.212 mol, 2 times the mol of diamantane) in order to avoid the run-away of reaction, followed by stirring at 30°C for 3 hours. The reaction mixture after 3 hours was in a suspension state. The reaction mixture was analyzed by GC. The result indicated that diamantane (a raw material) remained by 74 mass %, there was formation of monochlorodiamantane, dichlorodiamantane and trichlorodiamantane by 2 mass %, 22 mass % and 2 mass %, respectively, and there was no proceeding of reaction. Thereto was added 37.1 g (0.318 mol, 3 times the mol of diamantane) of chlorosulfonic acid (the total addition amount of chlorosulfonic acid: 5 times the mol of diamantane), and stirring was conducted at 30°C for 15 hours (the total reaction time: 18 hours). Analysis by GC indicated that the remaining diamantane was not found and there was formation of monochlorodiamantane, dichlorodiamantane and trichlorodiamantane by 6 mass %, 86 mass % and 8 mass %, respectively. The reaction mixture was cooled to about 10°C and then 44 g of water was dropwise added thereto so that the temperature of the reaction mixture did not exceed 30°C. 140 g of methylene chloride was added, followed by stirring. The mixture was allowed to stand and then the aqueous sulfuric acid solution layer (which was a lower layer) was separated. Then, the organic layer was washed total three times, that is, one time with 40 g of a 10 mass % aqueous sodium hydroxide solution and two times with 40 g and 20 g of a 7 mass % aqueous sodium sulfate solution, whereby the pH of the organic layer became neutral. The organic layer was concentrated under reduced pressure to obtain 28 g of a cream-colored solid (containing dichlorodiamantane by 82%). Yield: 85% (relative to diamantane)

The dichlorodiamantane obtained was 4,9-dichlorodiamantane almost selectively.

### Examples 2 to 4

An operation was conducted in the same manner as in Example 1 except that the amount of chlorosulfonic acid used was changed as shown in Table 1. The results are shown in Table 1.

**Table 1**

| Example | Chlorosulfonic acid (Mol times) | | 4,9-Dichlorodiamantane | | |
|---|---|---|---|---|---|
| | 1st time | 2nd time | Yield (g) | Yield (%) | Purity (%) |
| 1 | 2 | 3 | 28 | 85 | 82 |
| 2 | 3 | 2 | 28 | 87 | 84 |
| 3 | 3 | 3 | 30 | 88 | 80 |
| 4 | 2.5 | 2.5 | 28 | 90 | 83 |

### Example 5

A reaction and a post-treatment were conducted in the same manner as in Example 1. To the crude substance obtained was added 20 g (1 time the mass of a raw material diamantane) of n-heptane, followed by 2 hours refluxing with heating. The reaction mixture was always in a suspension state. Then, the reaction mixture was cooled to 5°C, stirred for 5 hours, aged, and filtered to obtain 22 g of a white solid (containing dichlorodiamantane by 96%) at a yield of 78% (relative to diamantane).

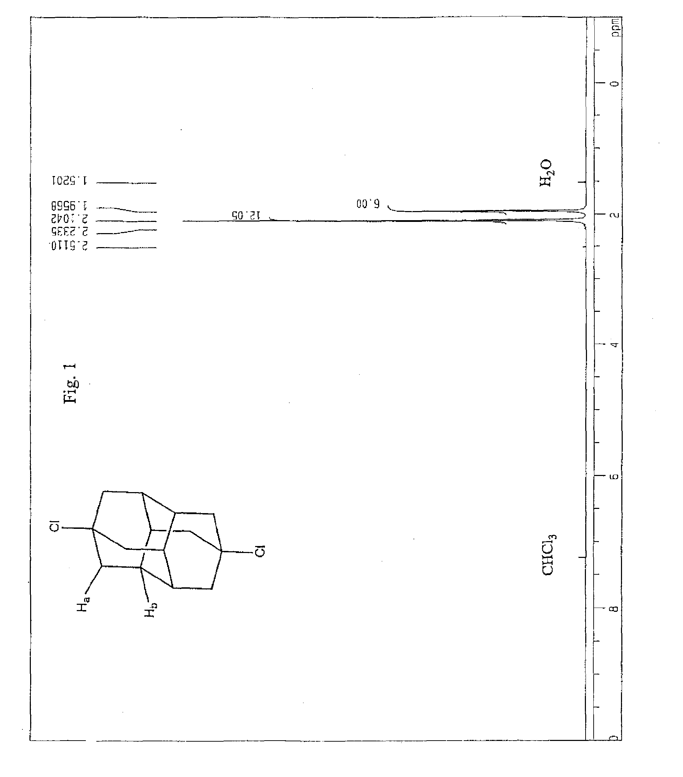

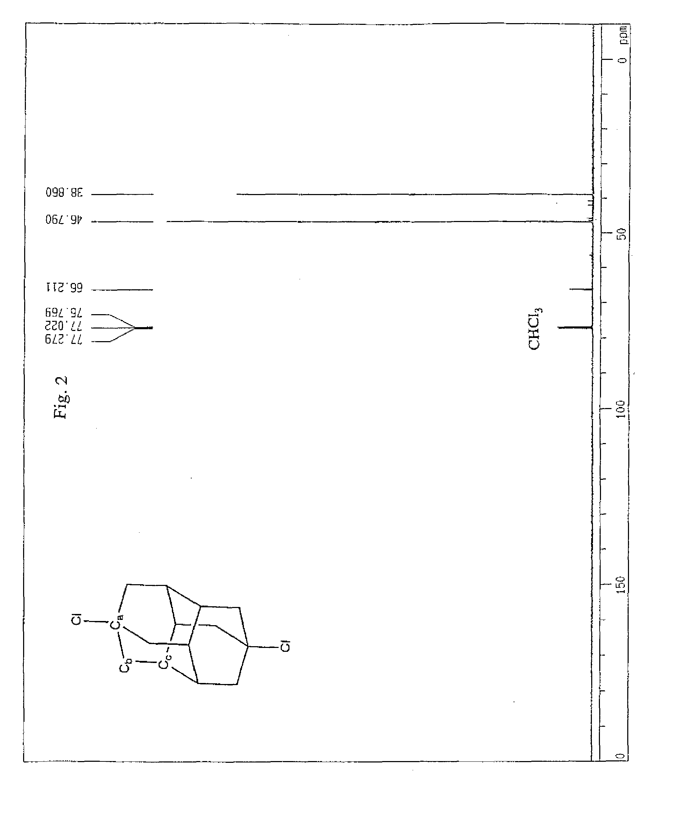

The dichlorodiamantane obtained was almost selectively 4,9-dichlorodiamantane. In Fig. 1 is shown a proton nuclear magnetic resonance (NMR) spectrum; in Fig. 2 is shown a carbon nuclear magnetic resonance (NMR) spectrum.
MASS (EI): molecular weight 256 (M⁺)
¹H-NMR spectrum (standard: TMS):
δ 2.10 (Hₐ, s, 12H), δ 1.96 (H_{b}, s, 6H)
¹³C-NMR spectrum (standard: TMS)
δ 66.2 (Cₐ), δ 46.7 (C_{b}), δ 38.9 (C_{c})

### Example 6

18.5 g (0.318 mol, 3 times the mol of diamantane) of sodium chloride and 20 g (0.106 mol) of diamantane were placed in a 1,000-ml, four-necked flask in a nitrogen current. Thereto was gradually added 123 g (1.06 mols, 10 times the mol of diamantane) of chlorosulfonic acid in order to avoid the run-away of reaction, followed by stirring at 30°C for 2 hours. The reaction mixture after the 2 hours was in a suspension state. The reaction mixture was analyzed by GC. The result indicated that diamantane (a raw material) remained by 78 mass %, there was formation of monochlorodiamantane, dichlorodiamantane and trichlorodiamantane by 2 mass %, 15mass % and 15 mass %, respectively, and there was no proceeding of reaction. Thereto was added 123 g (0.106 mol, 10 times the mol of diamantane) of chlorosulfonic acid (the total addition amount of chlorosulfonic acid: 20 times the mol of diamantane), and stirring was conducted at 60°C for 10 hours (the total reaction time: 12 hours). After the stirring of 10 hours, analysis by GC was conducted.

The analysis indicated that there was formation of dichlorodiamantane and trichlorodiamantane by 12 mass % and 88 mass %, respectively. The reaction mixture was cooled to 10°C and then 160 g of water was dropwise added thereto so that the temperature of the reaction mixture did not exceed 30°C. 200 g of methylene chloride was added, followed by stirring. The mixture was allowed to stand and then the aqueous sulfuric acid solution layer (which was a lower layer) was separated. Then, the organic layer was washed total three times, that is, one time with 40 g of a 10 % aqueous sodium hydroxide solution and two times with 40 g and 20 g of a 7 % aqueous sodium sulfate solution, whereby the pH of the organic layer became neutral. The organic layer was concentrated under reduced pressure to obtain 30 g of a cream-colored solid (containing trichlorodiamantane by 88 mass %). Yield: 86% (relative to diamantane)

The trichlorodiamantane obtained was 1,4,9-trichlorodiamantane almost selectively.

### Example 7

In a 2,000-ml, four-necked flask were placed, in a nitrogen current, 18.5 g (0.318 mol, 3 times the mol of diamantane) of sodium chloride and 20 g (0.106 mol) of diamantane. Thereto were gradually added 74.1 g (0.636 mol, 6 times the mol of diamantane) of chlorosulfonic acid in order to avoid the run-away of reaction. The mixture was stirred at 30°C for 2 hours. The reaction mixture was analyzed by GC. The result indicated that the diamantane as a raw material remained by 78 mass %, there was formation of monochlorodiamantane, dichlorodiamantane and trichlorodiamantane by 2 mass %, 18 mass % and 2 mass %, respectively, and there was no proceeding of reaction. Thereto was added 74.1 g (0.636 mol, 6 times the mol of diamantane) of chlorosulfonic acid, followed by stirring at 30°C for 2 hours. The reaction mixture was analyzed by GC. The result indicated that the diamantane as a raw material remained by 15 mass %, there was formation of monochlorodiamantane, dichlorodiamantane and trichlorodiamantane by 2 mass %, 70 mass % and 13 mass %, respectively, and there was no proceeding of reaction.

Thereto was added 74.1 g (0.636 mol, 6 times the mol of diamantane) (in total, 18 times the mol of diamantane) of chlorosulfonic acid, followed by stirring at 40°C for 24 hours (a reaction of total 28 hours was conducted). After the 24 hours stirring, the reaction mixture was analyzed by GC. The result indicated that dichlorodiamantane and trichlorodiamantane were formed by 12 mass % and 88 mass %.

The reaction mixture was cooled to 10°C. Then, 540 g of water was dropped into the reaction mixture while the temperature of the reaction mixture was being kept so as not to exceed 30°C. 1,000 g of methylene chloride was added to the reaction mixture, followed by stirring. The mixture was allowed to stand. Then, the aqueous sulfuric acid solution (which was a lower layer) was separated. Thereafter, the organic layer was washed total five times, that is, one time with 100 g of a 10% aqueous sodium hydroxide solution, total two times with 100 g of a 7% aqueous sodium sulfate solution and total 2 times with 100 g of ion-exchanged water. As a result, the pH of the organic layer was neutral. The organic layer was concentrated under reduced pressure to obtain 30 g of a cream-colored solid (containing of trichlorodiamantane by 88 mass %) of a cream-colored solid. Yield: 86% (relative to diamantane).

The trichlorodiamantane obtained was 1,4,9-trichlorodiamantane almost selectively.

### Example 8

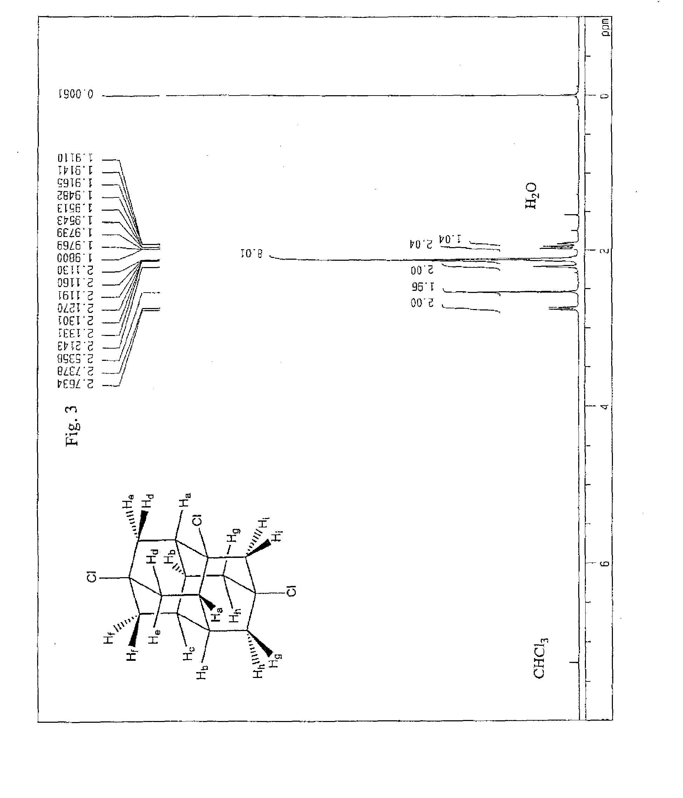

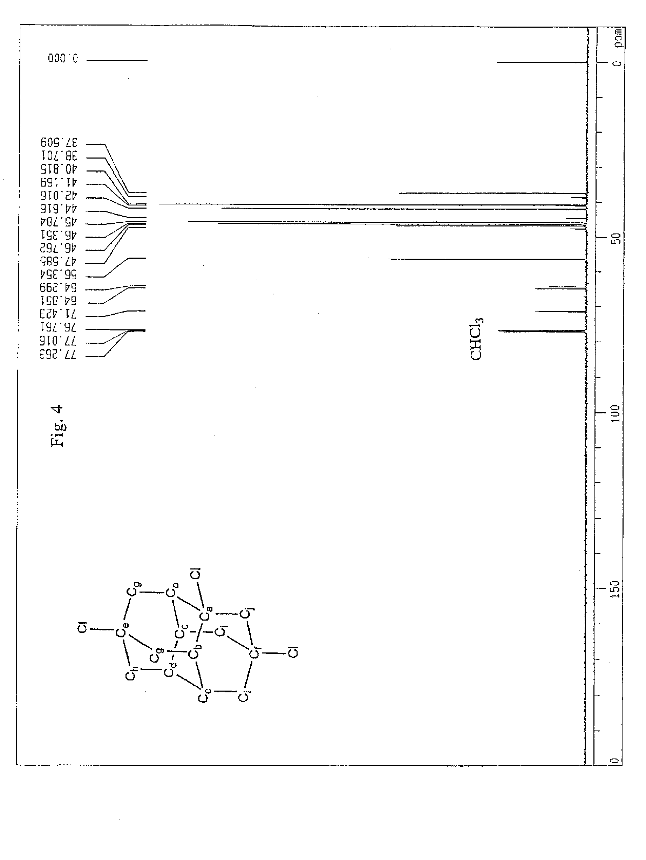

A reaction and a post-treatment were conducted in the same manner as in Example 6. To the crude trichlorodiamantane obtained were added 150 g (5 times the weight of crude trichlorodiamantane) of n-heptane and 90 g (3 times the weight of crude trichlorodiamantane) of chloroform. The mixture was refluxed with heating, for 1 hour. After the 1 hour refluxing with heating, the crude trichlorodiamantane was in a dissolved state, indicating a uniform solution. Then, the solution was cooled to 5°C, followed by stirring for 1 hour and aging. Thereafter, the reaction mixture was filtered to obtain 19.6 g of a white solid (containing trichlorodiamantane by 95%) at a yield of 61% (relative to diamantane). In Fig. 3 is shown a proton nuclear magnetic resonance (NMR) spectrum; in Fig. 4 is shown a carbon nuclear magnetic resonance (NMR) spectrum. It is appreciated from these spectra that the trichlorodiamantane produced by the present method is 1,4,9-trichlorodiamantane almost selectively.
MASS (EI): molecular weight 290 (M⁺)
¹H-NMR spectrum (standard: TMS):
δ 1.91 ~ δ 1.92 (H_{c}, m, 1H),
δ 1.95~δ 1.98 (Hₑ, d, 2H),
δ 2 .11~ δ2 . 13 (H_{b}, H_{f}, H_{g}, Hₕ, m, 8H),
δ 2.21 (Hₐ, s, 2H) , δ 2.54 (Hᵢ, s, 2H),
δ 2.74 ~ δ 2.76 (H_{d}, d, 2H)
¹³C-NMR spectrum (standard: TMS):
δ 37.5 (C_{d}), δ 40.8 (C_{b}), δ 42.0 (Cᵢ),
δ 45.8 (C_{g}), δ 46.4 (C_{c}), δ 46.7 (Cₕ),
δ 56.4 (Cⱼ), δ 64.3, 64.9 (Cₑ, C_{f}),
δ 71.4 (Cₐ)

### Examples 9 to 11

An operation was conducted in the same manner as in Example 1 except that the amount of sodium chloride used in Example 8 was changed as shown in Table 2. Each solid obtained was observed in color. The results are shown in Table 2.

**Table 2.**

| Example | Sodium chloride (mol times) | Trichlorodiamantane | | | |
|---|---|---|---|---|---|
| | | Yield (g) | Yield (%) | Purity (%) | Color |
| 9 | 0 | 32 | 94 | 90 | Light brown |
| 10 | 0.1 | 30 | 88 | 90 | Yellow |
| 11 | 1 | 30 | 87 | 89 | White |

### Example 12

In a 500-ml, four-necked flask were placed, in a nitrogen current, 1.5 g (0.0106 mol, 0.1 time the mol of diamantane) of sodium sulfate and 74.1 g (0.636 mol, 6 times the mol of diamantane) of chlorosulfonic acid. The four-necked flask inside was cooled to 5°C. 20 g (0.106 mol) of diamantane was gradually added in order to avoid the run-away of reaction. While the flask inside was being kept at 5°C, stirring was conducted for 8 hours. The reaction mixture after the 8 hours was in a suspension state. The reaction mixture was analyzed by GC. The result indicated that the presence of diamantane (a raw material) was not confirmed and there was formation of 4 mass % of monochlorodiamantane, 81 mass % of dichlorodiamantane and 15 mass % of trichlorodiamantane.

The reaction mixture was cooled to about 5°C and 30 g of water was dropped with care being taken so that the temperature of the reaction mixture did not exceed 30°C. 140 g of methylene chloride was added, followed by stirring. The mixture was allowed to stand. The resulting lower layer (an aqueous sulfuric acid solution layer) was separated. The organic layer was washed total three times, that is, one time with 40 g of a 10 mass % aqueous sodium hydroxide solution and two times with 40 g and 20 g of a 7 mass % aqueous sodium sulfate solution. As a result, the pH of the organic layer became neutral. The organic layer was concentrated under reduced pressure to obtain 28 g of a cream-colored solid (containing dichlorodiamantane by 80%). Yield: 82% (relative to diamantane).

The dichlorodiamantane obtained was 4,9-dichlorodiamantane almost selectively.

### Example 13

In a 500-ml, four-necked flask were placed, in a nitrogen current, 40 g (2 times the mass of diamantane) of concentrated sulfuric acid and 1.5 g (0.0106 mol, 0.1 time the mol of diamantane) of sodium sulfate. Further, 20 g (0.106 mol) of diamantane was added. The mixture was stirred for 10 minutes while being kept in a suspension state at about 20°C. Thereto was added 61.8 g (0.53 mol, 5 times the mol of diamantane) of chlorosulfonic acid gradually in order to avoid the run-away of reaction. A longer addition time than in Example 1 was needed because the chlorosulfonic acid reacted fairly violently at the time of its addition. Then, stirring was conducted at 30°C for 18 hours. After the 18 hours, the reaction mixture was analyzed by GC. As a result, diamantane disappeared and was not found and there was formation of 7 mass % of monochlorodiamantane, 86 mass % of dichlorodiamantane and 7 mass % of trichlorodiamantane.

The reaction mixture was cooled to about 10°C and 44 g of water was dropped with care being taken so that the temperature of the reaction mixture did not exceed 30°C. 140 g of methylene chloride was added, followed by stirring. The mixture was allowed to stand. The resulting lower layer (an aqueous sulfuric acid solution layer) was separated. The organic layer was washed total three times, that is, one time with 40 g of a 10 mass % aqueous sodium hydroxide solution and two times with 40 g and 20 g of a 7 mass % aqueous sodium sulfate solution. As a result, the pH of the organic layer became neutral. The organic layer was concentrated under reduced pressure to obtain 28 g of a cream-colored solid (containing dichlorodiamantane by 820). Yield: 85% (relative to diamantane).

The dichlorodiamantane obtained was 4,9-dichlorodiamantane almost selectively.

### Examples 14 to 16

An operation was conducted in the same manner as in Example 1 except that the diamantane used in Example 1 was changed as shown in Table 3. The results are shown in Table 3.

**Table 3**

| | Diamantane | | Dichlorodiamantane | | |
|---|---|---|---|---|---|
| Example | Compound used | Amount used (g) | Yield (g) | Yield (%) | Purity (%) |
| 14 | 1-Methyldiamantane | 21.4 | 31 | 94 | 84 |
| 15 | 1-Ethyldiamantane | 22.9 | 35 | 94 | 81 |
| 16 | 4,9-Dimethyldiamantane | 22.9 | 33 | 91 | 83 |

### Comparative Example 1

1.5 g (8 mmol) of diamantane was placed in a 100-ml, four-necked flask in a nitrogen current. The flask inside was cooled to 0°C. In this state of 0°C, 4 ml of bromine was added, and the mixture was cooled to -7°C. Then, 0.04 g (0.15 mmol) of aluminum tribromide was added, followed by stirring for 30 minutes. After the 30 minutes, diamantane disappeared and there was formation of dibromoadmantane of 90%. The reaction mixture was dropped into 30 g of a 10% aqueous sodium hydrogensulfite solution. Then, extraction with 30 g of ether was conducted. The ether layer was washed with 30 g of ion-exchanged water and then concentrated to obtain 2.5 g of a cream-colored solid (containing dibromodiamantane by 900). Yield: 81% (relative to diamantane)

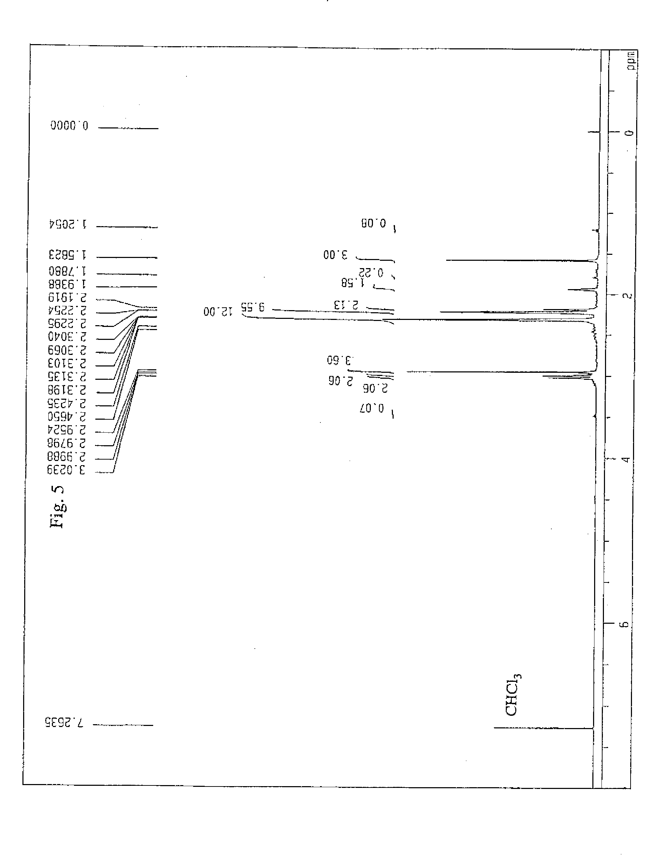

In Fig. 5 is shown a proton nuclear magnetic resonance (NMR) spectrum. As is clear from the spectrum of Fig. 5, the dibromodiamantane obtained contained not only 4,9-dibromdiamantane but also large amounts of 1,6-dibromodiamantane and 1,4-dibromodiamantane, and no dibromodiamantane of single structure was obtained.

### Example 17

In a glass-made autoclave (having an internal volume of 250 ml) were placed 28 g (0.109 mol) of the 4,9-dichlorodiamantane produced in Example 1, 17.9 g (0.218 mol, 2 times the mol of 4,9-dichlorodiamantane) of sodium acetate, 15.9 g (0.218 mol, 2 times the mol of 4,9-dichlorodiamantane) of N,N-dimethylformamide and 70 g (3.89 mols, 35.7 times the mol of 4,9-dichlorodiamantane) of ion-exchanged water. The mixture was heated to 150°C in a closed state. Then, stirring was conducted for 21 hours at 150°C at 0.4 MPa. After the 21 hours, the autoclave was cooled to room temperature and the contents were taken out. The contents were filtered to obtain 22 g of a light brown solid (the purity of 4,9-diamantanediol: 98%) of a light brown solid at a yield of 92%. Then, there was added, as a re-slurring solvent, 54 g (2 times the mass of 4,9-dichlorodiamantane used) of ethyl acetate, followed by stirring at 55°C for 1 hour. During the stirring, the 4,9-diamantanediol was not dissolved completely but was in a suspension state. Then, the mixture was cooled to 5°C, stirred for 2 hours and aged, then filtered to obtain 20 g (yield: 83%) of a white solid. The solid was analyzed by GC. As a result, the purity of 4,9-diamantanediol was 99%.

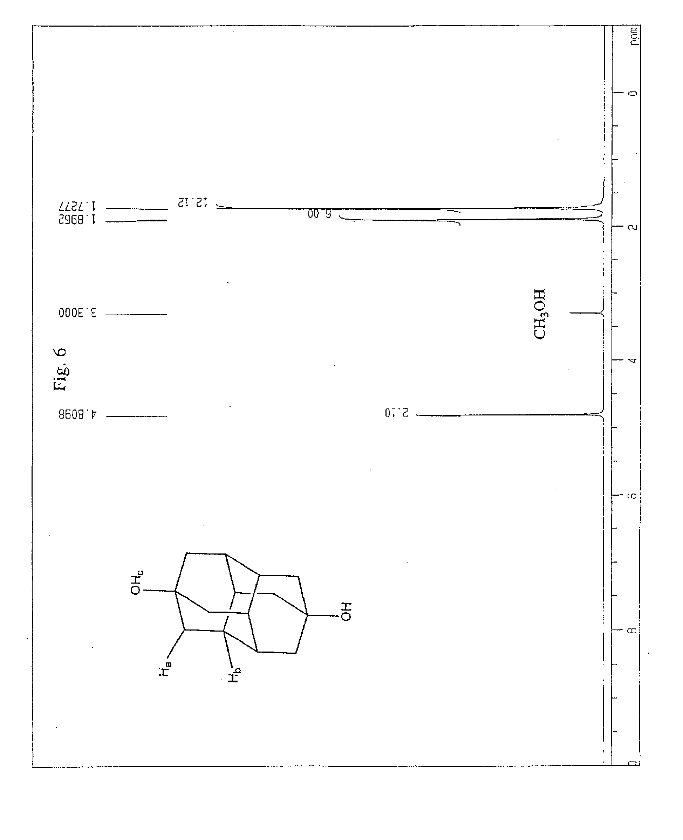

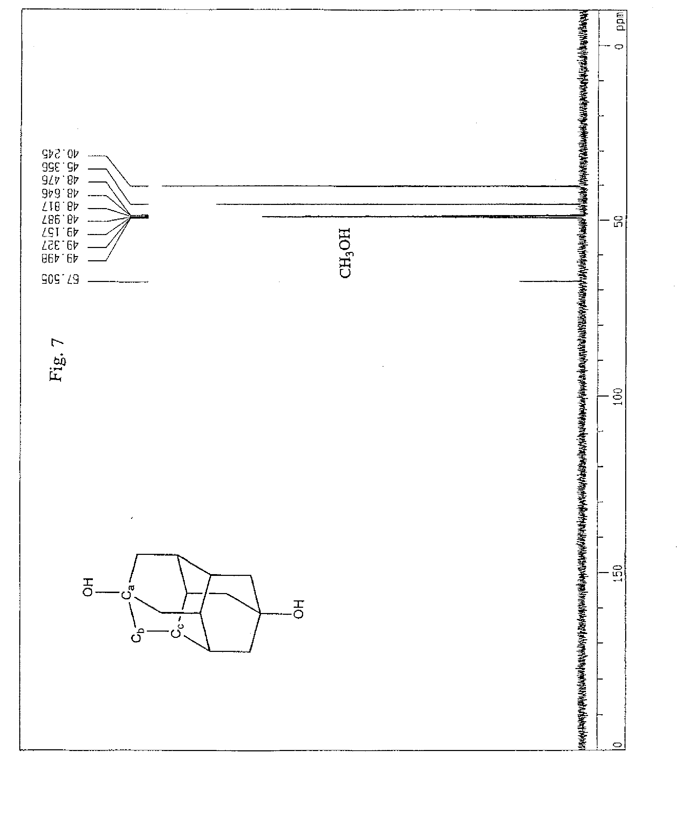

In Fig. 6 is shown a proton nuclear magnetic resonance (NMR) spectrum; in Fig. 7 is shown a carbon nuclear magnetic resonance (NMR) spectrum.
MASS (EI): molecular weight 220 (M⁺)
¹H-NMR spectrum (standard: methanol):
δ 1.73 (Hₐ, s, 12H), δ 1.90 (H_{b}, s, 6H),
δ 4.81 (H_{c}, s, 2H)
¹³C-NMR spectrum (standard: methanol):
δ 67.5 (Cₐ), δ 45.4 (C_{b}), δ 40.3 (C_{c})

### Examples 18 to 20

An operation was conducted in the same manner as in Example 17 except that the re-slurrying solvent used in Example 17 was changed to a solvent shown in Table 4. The results are shown in Table 4.

**Table 4**

| Example | Re-slurrying solvent | | 4,9-Diamantanediol | | |
|---|---|---|---|---|---|
| | Kind | Amount used | Yield (g) | Yield (%) | GC purity (%) |
| 18 | Acetone | 2 mass times | 20 | 83 | 99 |
| 19 | Acetonitrile | 2 mass times | 19 | 79 | 99 |
| 20 | Methylene chloride | 3 mass times | 20 | 83 | 99 |

### Examples 21 to 22

An operation was conducted in the same manner as in Example 17 except that the sodium acetate used in Example 17 was changed to a carboxylic acid salt shown in Table 5. The results are shown in Table 5.

**Table 5**

| Example | Carboxylic acid salt | | 4,9-diamantanediol | | |
|---|---|---|---|---|---|
| | Kind | Amount used | Yield (g) | Yield (%) | GC purity (%) |
| 21 | Lithium acetate | 2 mol times | 19 | 79 | 99 |
| 22 | Potassium acetate | 2 mol times | 20 | 83 | 99 |

### Example 23

In a glass-made autoclave (having an internal volume of 250 ml) were placed 20 g (0.069 mol) of the 1,4,9-trichlorodiamantane produced in Example 8, 17 g (0.207 mol, 3 times the mol of 1,4,9-trichlorodiamantane) of sodium acetate, 15.2 g (0.207 mol, 3 times the mol of 1,4,9-trichlorodiamantane) of N,N-dimethylformamide, 100 g (5.65 mols, 81 times the mol of 1,4,9-trichlorodiamantane) of ion-exchanged water. The mixture was heated to 150°C in a closed state, followed by stirring for 21 hours at 150°C at 0.4 MPa. After the 21 hours, the autoclave inside was cooled to room temperature and the contents were taken out. The contents were concentrated under reduced pressure to obtain a wet material containing 1,4,9-diamantanetriol. To the wet material were added 60 g of ion-exchanged water and 150 g of n-butanol, followed by stirring at 90°C for 1 hour. At the start of the stirring, the mixture was in a suspension state caused by 1,4,9-diamantanetriol but, 1 hour later, became a uniform solution. Then, the n-butanol layer was concentrated under reduced pressure to obtain 16 g of a light brown solid (the purity of diamantanetriol: 93%). Yield: 91%

Then, there was added, as a re-slurrying solvent, 54 g (2 times the mass of the 1,4,9-trichlorodiamantane used) of ethyl acetate, followed by stirring at 55°C for 1 hour. During the stirring, the 1,4,9-diamantanetriol was not dissolved completely but was in a suspension state. Then, the suspension was cooled to 5°C and stirring was conducted for 2 hours, aged, and filtered to obtain 14 g (yield: 86%) of a white solid. The solid was analyzed by GC. As a result, the purity of 1,4,9-diamantanetriol was 99%.

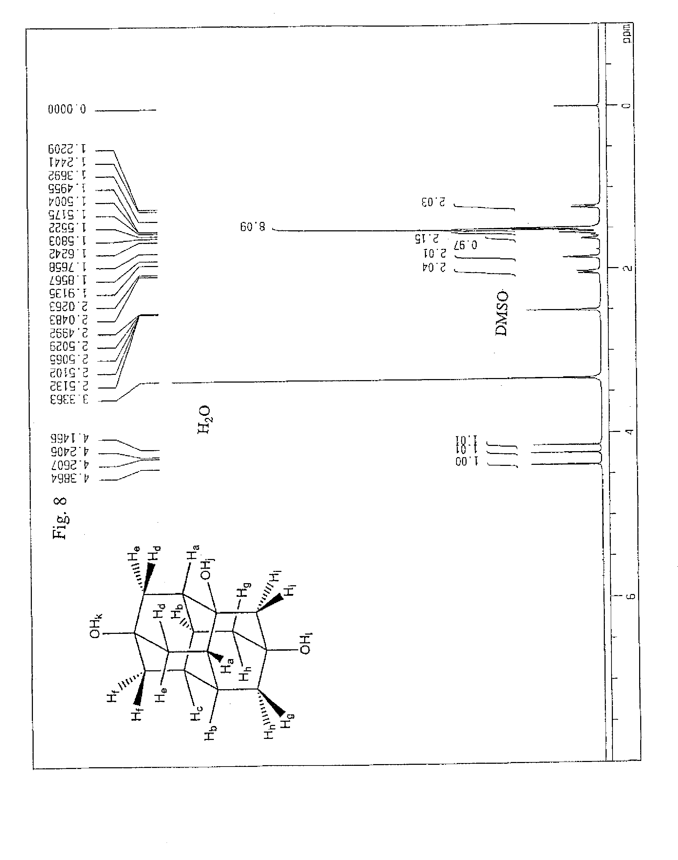

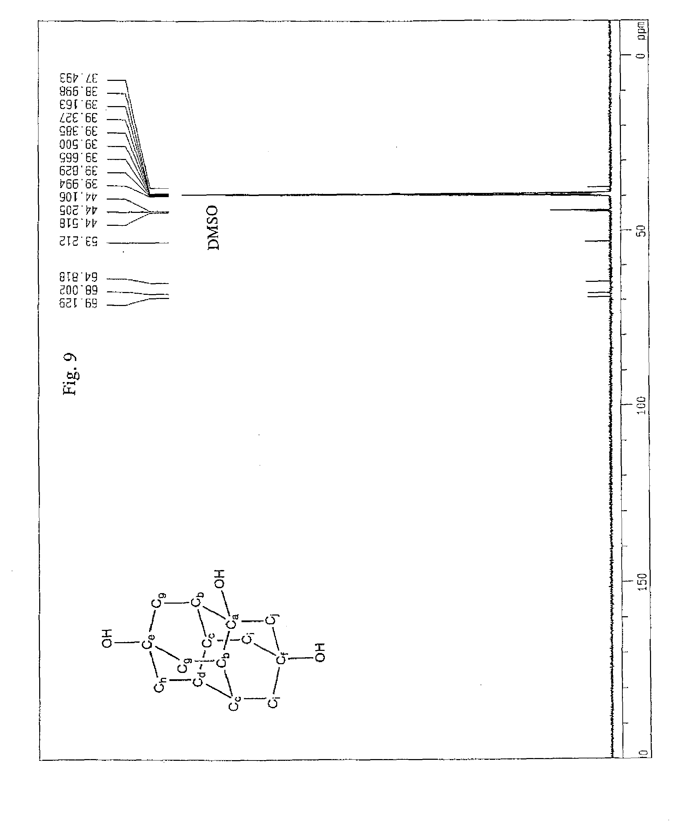

In Fig. 8 is shown a proton nuclear magnetic resonance (NMR) spectrum; in Fig. 9 is shown a carbon nuclear magnetic resonance (NMR) spectrum.
MASS (EI): molecular weight 236 (M⁺)
¹H-NMR spectrum (standard: TMS):
δ 1.22~1.24 (Hₑ, d, 2H),
δ 1.50~1.52 (H_{f}, H_{g}, Hₕ, Hᵢ, m, 8H),
δ 1.55 (Hₐ, s, 2H), δ 1.62 (H_{c}, s, 1H),
δ 1.86 (H_{b}, s, 2H), δ 2.03~2.05 (H_{d}, d, 2H),
δ 4.15, 4.24, 4.39 (Hⱼ, Hₖ, Hₗ, s, 3H),
¹³C-NMR spectrum (standard: TMS):
δ 37.5 (C_{d}) , δ 38.9-40.0 (C_{c}, C_{g}), δ 44.1 (Cᵢ),
δ 44.2 (C_{b}), δ 44.5 (Cₕ), δ53.2 (Cⱼ),
δ 64.8, 68.0, 69.1 (Cₐ, Cₑ, C_{f})

### Examples 24 to 26

An operation was conducted in the same manner as in Example 23 except that the re-slurrying solvent used in Example 23 was changed to a solvent shown in Table 6. The results are shown in Table 6.

**Table 6**

| Example | Re-slurrying solvent | | Diamantanetriol | | |
|---|---|---|---|---|---|
| | Kind | Amount used | Yield (g) | Yield (%) | GC purity (%) |
| 24 | Acetone | 2 mass times | 14 | 86 | 99 |
| 25 | Acetonitrile | 2 mass times | 14 | 86 | 99 |
| 26 | Methylene chloride | 3 mass times | 15 | 92 | 99 |

### Examples 27 to 28

An operation was conducted in the same manner as in Example 23 except that the sodium acetate used in Example 23 was changed to a carboxylic acid salt shown in Table 7. The results are shown in Table 7.

**Table 7**

| Example | Carboxylic acid salt | | Diamantanetriol | | |
|---|---|---|---|---|---|
| | Kind | Amount used | Yield (g) | Yield (%) | GC purity (%) |
| 27 | Lithium acetate | 2 mol times | 14 | 86 | 99 |
| 28 | Potassium acetate | 2 mol times | 14 | 86 | 99 |

### Comparative Example 2

In a glass-made autoclave (having an internal volume of 250 ml) were placed 28 g (0.109 mol) of 4,9-dichlorodiamantane, 17.9 g (0.218 mol, 2 times the mol of 4,9-dichlorodiamantane) of sodium acetate and 70 g (3.89 mols, 35.7 times the mol of 4,9-dichlorodiamantane) of ion-exchanged water. They were heated to 150°C in a closed state. Then, stirring was conducted for 21 hours at 150°C at 0.4 MPa. After the 21 hours, the autoclave was cooled to room temperature and the contents were taken out. At this time, there was, on the upper inner wall of autoclave, adhesion of a large amount of 4,9-diamantanediol, which presumably took place by sublimation. The later operation was conducted in the same manner as in Example, to obtain a solid. The solid was analyzed by GC. As a result, there was found no intended 4,9-diamantanediol that further isolation of the compound was not conducted.

### Comparative Example 3

In a glass-made autoclave (having an internal volume of 250 ml) were placed 28 g (0.109 mol) of 4,9-dichlorodiamantane, 15.9 g (0.218 mol, 2 times the mol of 4,9-dichlorodiamantane) of N,N-dimethylformamide and 70 g (3.89 mols, 35.7 times the mol of 4,9-dichlorodiamantane) of ion-exchanged water. They were heated to 150°C in a closed state. As a result, the pressure increased rapidly to about 1 MPa and the reaction was stopped. After cooling, the reactor inside was examined, which indicated the coloring of the reaction mixture in a black color.

## Claims

1. A method for producing a polyhalogenated diamantane, **characterized by** reacting a diamantane with a halosulfonic acid.

2. A method for producing a polyhalogenated diamantane according to Claim 1, wherein the polyhalogenated diamantane is a polychlorinated diamantane.

3. A method for producing a polyhalogenated diamantane according to Claim 2, wherein the polychlorinated diamantane is a 4,9-dichlorinated diamantane.

4. A method for producing a polyhalogenated diamantane according to Claim 2, wherein the polychlorinated diamantane is a 1,4,9-trichlorinated diamantane.

5. A method for producing a polyhalogenated diamantane according to Claim 1, wherein the reaction is conducted in the presence of concentrated sulfuric acid.

6. A method for producing a polyhalogenated diamantane according to Claim 1, wherein the reaction is conducted in the presence of an inorganic salt.

7. A method for producing a polyhalogenated diamantane according to Claim 6, wherein the inorganic salt is a sulfonic acid salt.

8. A method for producing a polyhalogenated diamantane according to any of Claims 1 to 7, wherein, in the reaction of the diamantane with the halosulfonic acid, the halosulfonic acid is divided into a plurality of fractions, one fraction thereof is first mixed with the diamantane for a reaction, and then the remaining fractions are each added to the diamantane successively when a prior reaction stops substantially, to conduct reactions a plurality of times.

9. A method for producing a diamantanepolyol, **characterized by** reacting a polyhalogenated diamantane produced by the method for producing a polyhalogenated diamantane, set forth in Claim 1, with water in the presence of a water-soluble organic solvent and a carboxylic acid salt.

10. A method for producing a diamantanepolyol according to Claim 9, wherein the polyhalogenated diamantane is a polychlorinated diamantane.

11. A method for producing a diamantanepolyol according to Claim 10, wherein the polychlorinated diamantane is a 4,9-dichlorinated diamantane.

12. A method for producing a diamantanepolyol according to Claim 10, wherein the polychlorinated diamantane is a 1,4,9-trichlorinated diamantane.

13. A method for producing a diamantanepolyol according to Claim 9, wherein the water-soluble organic solvent is N,N-dimethylformamide.

14. A method for producing a diamantanepolyol according to Claim 9, wherein the carboxylic acid salt is at least one member selected from the group consisting of lithium acetate, sodium acetate and potassium acetate.

15. A method for producing a diamantanepolyol according to Claim 9, wherein the reaction is conduced under pressure.

16. A method for producing a diamantanepolyol, **characterized by** forming a slurry using a diamantanepolyol obtained by the production method set forth in any of Claims 9 to 15 and a poor solvent for the diamantanepolyol, and then filtering the slurry to obtain a diamantanepolyol.

17. A method for producing a diamantanepolyol according to Claim 16, wherein the poor solvent is at least one member selected from the group consisting of esters and halogenated aliphatic hydrocarbons.
